# EUROPEAN PATENT SPECIFICATION

(11) **EP 1 524 920 B1**
(45) Date of publication and mention of the grant of the patent: **09.08.2006**
(21) Application number: 03762947.4
(22) Date of filing: 27.06.2003
(51) Int. Cl.: A41D 13/12, A41D 13/005, A61F 7/00, A61B 19/08

(54) **HEAT-EMITTING PATIENT GARMENT**
WÄRMEABGEBENDES PATIENTENKLEIDUNGSSTÜCK
VETEMENT A EMISSION DE CHALEUR POUR PATIENT

(30) Priority: 03.07.2002 SE 0202079
(43) Date of publication of application: 27.04.2005
(73) Proprietor: Mölnlycke Health Care AB, 402 52 Göteborg (SE)
(72) Inventor: MALMBERG, Angelica, S-434 00 Kungsbacka (SE); RAUK BERGSTRÖM, Tina, S-421 65 Västra Frölunda (SE); GELLERSTEDT, Fredrik, S-439 94 Onsala (SE)
(74) Representative: Hyltner, Jan-Olof
(86) International application number: PCT/SE2003/001132
(87) International publication number: WO 2004/004500

(56) References cited:
- WO-A1-00/62726
- FR-A1- 2 810 875
- US-A- 1 489 046
- US-A- 3 507 321
- US-A- 3 738 367
- US-A- 5 383 918
- US-A- 5 405 370
- US-A- 5 735 890
- US-A- 6 012 179

## Description

### TECHNICAL FIELD

This invention relates to a patient garment comprising a front piece and a back piece.

### BACKGROUND TO THE INVENTION

In most longer operations it is common for hypothermia, i.e. the body temperature is below 36 °C, to occur. Complications of hypothermia are decreased cardiac activity, arrhythmias, impaired blood coagulation, cardiac infarction, respiratory problems, stroke, haemorrhage and tremor. When normal temperature-regulating functions are inhibited the patient becomes dependent on the ambient temperature. In acute hypothermia there is vasoconstriction and the blood is taken from the skin to the internal organs in order to prevent further heat loss. If the vasoconstriction is prolonged, anaemia arises in the tissues. A compensating mechanism, known as the Lewis phenomenon, allows periodical vasoconstriction in order to provide the skin and external parts with oxygen. This mechanism is impeded if the temperature continues to fall.

It has been found that hypothermia can be prevented by warming the surface parts of the body by means of warm-air blankets (see US-A-5 405 370). Such blankets have been found to be the best commercially available alternative for maintaining the patient's body temperature during surgical operations.

It has also been found that it is advantageous to warm the surface parts of the body both before and after an operation.

There are today no warm-air blankets which are intended to be used before, during and after an operation, but instead different warming systems are used. The different warming systems are left in the same place while the patient is being moved between the ward and operating theatre. The patient is often naked or inadequately covered under the blankets, which means that movements between the wards and the operating theatre, during which the patient can be seen by the general public, can constitute a slight to the patient's dignity.

It is the object of this invention to produce a warming device for patients which can be used both before, during and after a surgical operation and which covers the patient during movements between the wards and the operating theatre.

### SUMMARY OF THE INVENTION

This object is achieved by means of a patient garment comprising a front piece and a back piece, characterized in that the front piece is wholly or partially made up of an outer layer of a material with little air permeability and an inner layer of a material with a significantly higher air permeability than the outer layer, which extends over at least part of the outer layer and is attached to the latter in such a way that a space is formed between the outer and inner layer, and that at least one inlet connectable to a source of warm air is located on the front piece of the garment and leads to the space between the inner and outer layer.

In a preferred embodiment the front and back pieces are detachably and reclosably connected with each other, at least in the area from the shoulder parts to the end of the sleeve, and the front piece comprises openable parts to allow access to the area for the operation. In a first variant the garment is designed as a coat, the front piece has a central slit which extends from the bottom end of the coat to the breast part thereof, and the parts of the front piece which extend along the edges of the slit are connected with each other by means of a detachable and reclosable connection. The coat has an openable back. In a second variant the garment is designed as a pyjama with a top part and a trouser part, and the front piece of the top part is connected with the back part by means of a detachable and reclosable connection, at least along the sides from the lower end of the top part to the breast part thereof. The back piece and the inner layer of the front piece are preferably made of an air-permeable non-woven material and the outer layer of the front piece is preferably made of a plastic film. The inner layer can advantageously extend over the whole of the outer layer and be laminated to the outer layer within the marginal area of the openable parts of the front piece. Furthermore, the detachable and reclosable connections which connect parts of the garment with each other are made up of mechanical connections, preferably so-called velcro connections.

### LIST OF FIGURES

The invention will now be described with reference to appended figures, of which;
Fig. 1 shows a schematic front view of a patient garment according to a first embodiment of the invention,
Fig. 2 shows a view of the same kind as Figure 1 of a patient garment according to a second embodiment of the invention,
Fig. 3 shows a section along the line III-III in Figure 1, and
Fig. 4 and 5 show views of the same kind as Figure 1 of a patient garment according to further embodiments of the invention.

### DESCRIPTION OF EMBODIMENTS

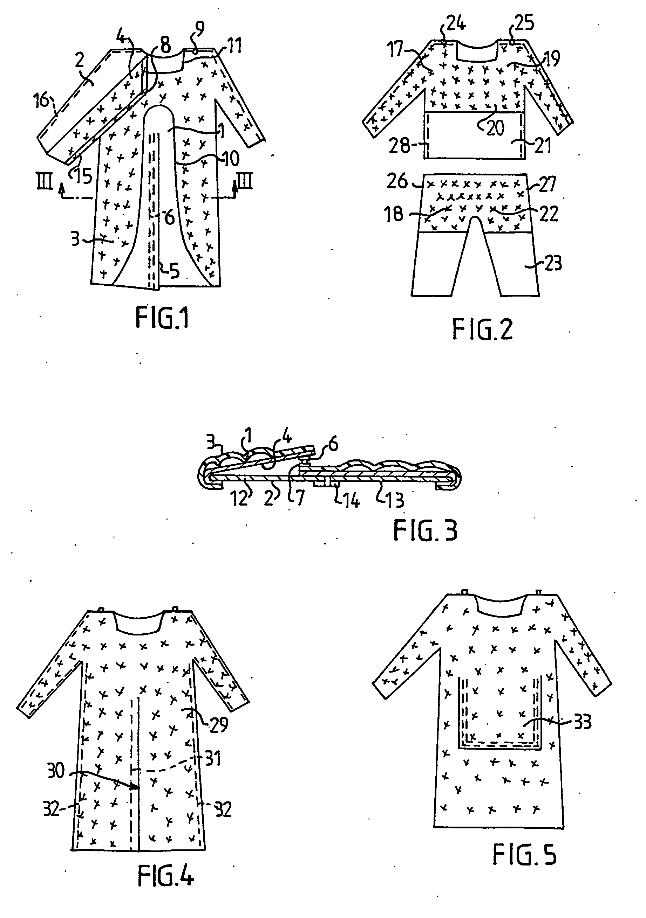

The patient garment shown in Figures 1 and 3 is designed as a coat with a front piece 1 and a back piece 2. The front piece comprises an outer layer 3 of a material with little or no air permeability and an inner layer 4 of a material with significantly higher air permeability than the outer layer. Furthermore, the front piece has a central slit 5 which extends from the bottom end of the front piece to level with the breast part. The parts of the front piece lying on either side of the slit are connected with each other in an overlapping manner by means of a detachable and reclosable connection, preferably a mechanical connection of the velcro type, i.e. a connection with a male element 6, which contains a number of protruding hook members, and a female element 7, which contains a number of loops or the like, in which the hook members engage when the elements are brought together. The inner layer 4 is connected with the outer layer 3 by means of a sparse pattern of adhesion or weld points over the greater part of the front piece. Furthermore, an air inlet 8,9 is attached to the front piece in each shoulder part, which inlet emerges between the inner and outer layer. The inlets which are not being used are closed in a suitable manner, e.g. the inlets can be made up of tubular bodies which are folded and sealed with adhesive patches or provided with simple paper or plastic seals. The inner and outer layer are hermetically connected with each other along the periphery of the front piece and along a line 10, which extends at some distance from the slit 5, and a line 11 around the neckband. Air which is introduced through either of the inlets 8 or 9 thus only has access to the space between the inner and outer layer in the area where these layers are connected with each other by means of a sparse pattern of adhesion or weld points.

The back piece 2 is preferably divided into two parts in order to make it easy to put the coat on and the two parts 12, 13 are held together by a detachable and reclosable connection 14, e.g. a velcro connection.

The front and back piece are detachably and reclosably connected with each other in the sleeve and shoulder parts in such a way that the front piece can be folded out of the way of the back piece within these parts in the manner which is shown in the left part of Figure 1. In this way the patient's arms can be bared for the application of monitoring equipment, such as blood pressure cuffs and needles. In this case also the detachable and reclosable connections can be made up of a male element 15 and female element 16 in a velcro connection.

The coat according to Figures 1 and 3 is intended to be put on a patient already in the ward where the latter is being prepared for operation. One of the air inlets 8,9 is then opened and connected to a source of warm air, e.g. a unit of the type used for warm-air blankets, such as Bairhugger Model 500/OR from Augustine Medical Inc., Eden Prairie, Minnesota, USA, Warmtouch Model 5200 from Nellcor, Pleasanton, California, USA or Thermacare 3000 from Gaymar, Orchard Park, New York, USA. Because the inner layer of the front piece is air-permeable, warm air can flow through the inner layer and warm up the patient's skin. The warming of the patient's skin before an operation should go on for at least an hour, as it has been found that warming for an hour considerably reduces the drop in body temperature during subsequent anaesthesia, see Camus et al. "Pre-Induction Skin-Surface Warming Minimizes Intraoperative Core Hypothermia", Journal of Clinical Anesthesia 7:384-388, 1995.

The patient is then taken to the operating theatre with the coat on. If there is no warm-air unit in the operating theatre, this is taken as well. When the patient has been laid on the operating table one of the patient's arms is exposed by opening the connection 15,16 and folding up the opened part of the front piece, and any monitoring equipment is connected to the arm and chest, after which the part of the front piece that has been folded up is folded back and selected parts of the connection 15,16 are reclosed. The area for operation is also exposed by undoing the connection 6,7 along the slit 5, folding the unfastened parts of the front piece out of the way in a suitable manner and attaching the parts that have been folded out of the way to the skin by means of fixing tape. The patient is then draped by means of a suitable draping system. The warm-air unit which is present in or brought to the operating theatre is connected up either immediately on the patient's arrival in the operating theatre or at the latest when draping is finished. During the subsequent surgical operation the patient's skin is thus warmed up in the area outside the area for operation, which considerably reduces the risk of hypothermia occurring.

After the surgical operation has been performed, the parts of the front piece that have been folded out of the way are detached and fastened to each other by means of the connection 6,7, after which the patient, with the coat on, is taken to the observation ward. During the stabilization phase after anaesthesia a warm-air unit is connected to the coat and warm air is supplied to the patient's skin.

Because the patient garment described above is worn by the patient both before and during and after a surgical intervention, the risk of post-operative infections due to hypothermia can be considerably reduced in a cost-effective manner, while at the same time ensuring that there is no slight to the patient's dignity during movements between wards and the operating theatre because of inadequate covering.

For the coat described above to function properly, the inner layer 4 of the front piece 1 must not have too high air permeability but warm air must first fill the space between the connection points in the area where the outer and inner layers are connected with each other with a sparse binding pattern before the warm air can escape from the inner layer and flow towards the skin of a patient. The material in the inner layer can be made up of perforated plastic films, with it being easy to adjust the perforation pattern of the plastic film in such a way that a suitable air permeability is obtained. It is also conceivable to use dense non-woven material of natural or synthetic fibres and even dense textile material. If a plastic film is used for the inner layer, this can advantageously be laminated to a non-woven material with higher air permeability than the perforated plastic film in order to increase the patient's comfort, especially when the patient garment is used without an influx of warm air.

The outer layer 3 of the front piece 1 can suitably be made up of a plastic film of polypropylene, polyethylene or polyester or other dense plastic materials. Other materials with little or no air permeability can also be used. Little air permeability in this application means an air permeability which is considerably lower than the air permeability of the inner layer.

The back piece 2 is suitably made of a soft and skin-friendly material, for example a spunlace non-woven, e.g. the material Sontara® from DuPont Nonwovens, Sontara® Technologies, Old Hickory, Tennessee, USA.

Figure 2 shows another embodiment of a patient garment according to the invention. This garment differs from the coat shown in Figures 1 and 3 mainly by the fact that it is designed as a pyjama with a top part 17 and a trouser part 18. The front piece 19 of the top part 17 has an upper part 20, in which an outer and an inner layer are connected with each other with a sparse pattern of adhesion or weld points, and a lower part 21, which is separated from the upper part by a continuous glued or welded seam. In the same way the front piece of the trouser part 18 has an upper area 22, in which an outer and an inner layer are connected with each other with a sparse pattern of adhesion or weld points, and a lower part 23, which is separated from the upper part 22 by a continuous glued or welded seam. Air inlets 24, 25 and 26,27 lead to the upper areas 20, 22 of the top part and trouser part, respectively. The front and the back piece of the top part 17 are detachably and reclosably connected with each other in the sleeve and shoulder parts in the same way as in the embodiment described with reference to Figures 1 and 3, in such a way that the front piece can be folded out of the way of the back piece within these parts in the manner which is shown in the left part of Figure 1. Furthermore, the lower part 21 of the front piece 19 is detachably and reclosably connected in the top part 17 with the back piece along its side edges by means of connection 28, a velcro connection for example. Part 21 can thus be rolled up or folded out of the way in order to expose the area for operation.

The patient garment shown in Figure 2 is made of the same material as the patient garment shown in Figures 1 and 3.

Figure 4 shows yet another patient garment, which constitutes a combination of the garments described earlier. The front piece 29 of the patient coat shown in Figure 4 thus includes a slit 30. The parts of the front piece lying on either side of the slit are connected with each other by means of a connection 31 corresponding to the connection 6,7 described in Figures 1 and 3. Furthermore, the side edges of the front piece from the bottom end to level with the end of the slit 30 are provided with a connection 32 corresponding to connection 28 in the patient garment shown in Figure 2. In addition to being able to produce operation apertures in the manner which has been described for the respective embodiments in Figures 1 and 2, operation apertures can be produced by rolling up that part of the front piece 29 which lies to the left or to the right, respectively, of the slit 30.

Figure 5 shows yet another embodiment of a patient garment according to the invention. This patient garment differs from the patient coat shown in Figure 1 and 4 by the fact that the coat does not have any slit. Instead, an operation aperture is produced by opening a flap 33 of front piece material. This flap is attached to the edges of an operation aperture by means of a velcro connection or the like. As is apparent from Figure 5, air can flow along the side of the flap 33 and down to the lowest part of the coat shown in Figure 5.

The embodiments described can of course be modified in a number of ways within the scope of the invention. The coat shown in Figure 1 can for example be provided with a part which can be rolled up in the same way as the two-part garment shown in Figure 2, instead of a slit. Furthermore, the inner layer of the front piece does not need to extend over the whole of the outer layer but can merely extend over the areas which are intended to emit warm air when connected to a warm-air unit. Those parts of the front piece which lie outside the space to which the warm-air inlets lead can consist of any suitable material whatsoever, e.g. of the outer layer, the inner layer, a laminate of the outer and inner layer or a third material. Furthermore, the spaces to which the warm-air inlets lead can have a different configuration to the one shown in the figures, e.g. they can extend over the whole area of the front piece. The detachable and reclosable connections can be made up of other connections than velcro connections, e.g. zip fasteners, press studs or adhesive connections, nor do they need to have the dimensions shown in the figures. It would be possible for example for the connection 6,7 in Figure 1 to be made up of one, two or more separate connections positioned at a distance from each other. Nor is there a need for two air inlets, instead it is sufficient for the garment to have one air inlet. Moreover, the pattern of weld or adhesion points can have a different appearance from the one shown in the figures, nor does the pattern need to consist of points but can consist of lines. Such lines must not be so long, however, that they do not allow entry of air from adjacent spaces delimited by lines between the outer and inner layers of the front piece. It is even possible to laminate a non-woven to the outside of the front piece in order to give the patient garment a more textile-like appearance. Furthermore, the lower part 21 of the front piece in the embodiment shown in Figure 2 can also be designed to emit warm air in the same way as the upper part 20, since the lower part can be hermetically separated from the lower part when the lower part is rolled up in order to produce an operation aperture. The scope of the invention will therefore only be limited by the content of the appended claims.

## Claims

1. Patient garment comprising a front piece (1) and a back piece (2), **characterized in that** the front piece (1) is wholly or partially made up of an outer layer (3) of a material with little air permeability and an inner layer (4) of a material with a significantly higher air permeability than the outer layer, which extends over at least part of the outer layer (3) and is attached to the latter in such a way that a space is formed between the outer and inner layer, and that at least one inlet (8,9) connectable to a source of warm air is located on the front piece of the garment and leads to the space between the inner and outer layer.

2. Patient garment according to Claim 1, **characterized in that** the front and back pieces (1,2) are detachably and reclosably connected with each other, at least in the area from the shoulder parts to the end of the sleeve.

3. Patient garment according to Claim 1 or 2, **characterized in that** the front piece (1) includes openable parts to allow access to the area for operation.

4. Patient garment according to Claim 3, **characterized in that** the garment is designed as a coat, that the front piece (1) has a central slit (5) which extends from the bottom end of the coat to its breast part, and that those parts of the front piece which extend along the edges of the slit are connected with each other by means of a detachable and reclosable connection (6,7).

5. Patient garment according to Claim 3, **characterized in that** the garment is designed as a pyjama with a top part (17) and a trouser part (18), and that the front piece (19) of the top part is connected with the back piece by means of a detachable and reclosable connection (28), at least along the sides from the lower end of the top part to the breast part thereof.

6. Patient garment according to any of Claims 1-5, **characterized in that** the inner layer (4) of the back piece (2) and the front piece (1) is made of an air-permeable non-woven and the outer layer (3) of the front piece is made of a non-air-permeable material, e.g. a plastic film or a laminate of plastic film and non-woven.

7. Patient garment according to any of Claims 1-6, **characterized in that** the inner layer (4) extends over the whole of the outer layer (3).

8. Patient garment according to any of Claims 3-7, **characterized in that** the inner layer (4) is laminated to the outer layer (3) within the marginal area of the openable parts of the front piece (1).

9. Patient garment according to any of Claims 2-8, **characterized in that** the detachable and reclosable connections (6,7 and 15,16) which connect parts of the garment with each other are made up of mechanical connections, preferably so-called velcro connections.

10. Patient garment according to any of Claims 4, 6-9, **characterized in that** the garment has an openable back.

## Patentansprüche

1. Patienten-Kleidungsstück, umfassend einen Vorderteil (1) und einen Hinterteil (2), **dadurch gekennzeichnet, dass** der Vorderteil (1) vollständig oder teilweise aus einer äußeren Lage (3) aus einem Material mit geringer Luftdurchlässigkeit und einer inneren Lage (4) aus einem Material mit einer signifikant höheren Luftdurchlässigkeit als die äußere Lage und die sich über wenigstens einen Teil der äußeren Lage (3) erstreckt und an letzterer derart angebracht ist, dass ein Raum zwischen der äußeren und inneren Lage gebildet ist, aufgebaut ist und dass wenigstens ein Einlass (8, 9), der mit einer Warmluftquelle verbindbar ist, auf dem Vorderteil des Kleidungsstücks angeordnet ist und zum Raum zwischen der inneren und äußeren Lage führt.

2. Patienten-Kleidungsstück nach Anspruch 1, **dadurch gekennzeichnet, dass** der Vorder- und Hinterteil (1, 2) lösbar und wiederverschließbar miteinander verbunden sind und zwar wenigstens im Bereich von den Schultern zum Ende der Ärmel.

3. Patienten-Kleidungsstück nach Anspruch 1 oder 2, **dadurch gekennzeichnet, dass** der Vorderteil (1) Teile umfasst, die geöffnet werden können, um Zugang zum Bereich für die Operation zu gestatten.

4. Patienten-Kleidungsstück nach Anspruch 3, **dadurch gekennzeichnet, dass** das Kleidungsstück als ein Mantel ausgestaltet ist, dass der Vorderteil (1) einen mittigen Schlitz (5) aufweist, der sich vom unteren Ende des Mantels zu seinem Brustteil erstreckt und dass solche Teile des Vorderteils, die sich entlang der Kanten des Schlitzes erstrecken, mittels einer lösbaren und wiederverschließbaren Verbindung (6, 7) verbunden sind.

5. Patienten-Kleidungsstück nach Anspruch 3, **dadurch gekennzeichnet, dass** das Bekleidungsstück als ein Pyjama mit einem Oberteil (17) und einer Hose (18) ausgestaltet ist und dass der Vorderteil (19) des Oberteils mittels einer lösbaren und wiederverschließbaren Verbindung (28) wenigstens entlang der Seiten vom unteren Ende des Oberteils zum Brustteil davon mit dem Hinterteil verbunden ist.

6. Patienten-Kleidungsstück nach einem der Ansprüche 1 bis 5, **dadurch gekennzeichnet, dass** die innere Lage (4) des Hinterteils (2) und des Vorderteils (1) aus einem luftdurchlässigen Vliesstoff aufgebaut ist und dass die äußere Lage (3) des Vorderteils aus einem luftdurchlässigen Material, z.B. einer Kunststofffolie oder einem Verbund aus einer Kunststofffolie und einem Vliesstoff aufgebaut ist.

7. Patienten-Kleidungsstück nach einem der Ansprüche 1-6, **dadurch gekennzeichnet, dass** sich die innere Lage (4) über die gesamte äußere Lage (3) erstreckt.

8. Patienten-Kleidungsstück nach einem der Ansprüche 3 bis 7, **dadurch gekennzeichnet, dass** die innere Lage (4) innerhalb des Randbereichs der Teile des Vorderteils (1), die geöffnet werden können, mit der äußeren Lage (3) verbunden ist.

9. Patienten-Kleidungsstück nach einem der Ansprüche 2 bis 8, **dadurch gekennzeichnet, dass** die lösbaren und wiederverschließbaren Verbindungen (6, 7 und 15, 16), die die Teile des Bekleidungsstücks miteinander verbinden, aus mechanischen Verbindungen, vorzugsweise sogenannten Klettverschlüssen, aufgebaut sind.

10. Patienten-Kleidungsstück nach einem der Ansprüche 4 und 6-9, **dadurch gekennzeichnet, dass** das Bekleidungsstück eine Rückseite aufweist, die geöffnet werden kann.

## Revendications

1. Un vêtement de patient comportant une pièce avant (1) et une pièce arrière (2), **caractérisé en ce que** la pièce avant (1) est entièrement ou partiellement fabriquée d'une couche extérieure (3) de matière à faible perméabilité à l'air et d'une couche intérieure (4) de matière à perméabilité à l'air distinctement plus élevée que la perméabilité à l'air de la couche extérieure, laquelle s'étend sur au moins une partie de la couche extérieure (3) et est attachée à cette dernière de manière à ce qu'un espace soit formé entre la couche extérieure et intérieure, et **en ce qu'**au moins une ouverture (8, 9) raccordable à une source d'air chaud est située sur la pièce avant du vêtement et mène à l'espace entre la couche intérieure et extérieure.

2. Un vêtement de patient selon la revendication 1, **caractérisé en ce que** les pièces avant et arrière (1, 2) sont reliées l'une à l'autre de manière détachable et refermable, au moins dans la région à partir des parties d'épaule jusqu'à l'extrémité de la manche.

3. Un vêtement de patient selon la revendication 1 ou 2, **caractérisé en ce que** la pièce avant (1) comprend des parties ouvrantes pour permettre d'accéder à la région lors de l'opération.

4. Un vêtement de patient selon la revendication 3, **caractérisé en ce que** le vêtement est conçu comme un manteau, **en ce que** la pièce avant (1) présente une fente centrale (5) laquelle s'étend à partir de l'extrémité de bas du manteau jusqu'à sa partie de poitrine, et **en ce que** ces parties de la pièce avant qui s'étendent le long des bords de la fente sont reliées l'une à l'autre au moyen d'une connexion détachable et refermable (6, 7).

5. Un vêtement de patient selon la revendication 3, **caractérisé en ce que** le vêtement est conçu comme un pyjama avec une partie de haut (17) et une partie de pantalon (18), et **en ce que** la pièce avant (19) de la partie de haut est reliée avec la pièce arrière au moyen d'une connexion détachable et refermable (28), au moins le long des bords à partir de l'extrémité inférieure de la partie de haut jusqu'à la partie de poitrine de celle-ci.

6. Un vêtement de patient selon l'une quelconque des revendications 1-5, **caractérisé en ce que** la couche intérieure (4) de la pièce arrière (2) et de la pièce avant (1) est faite d'un nontissé perméable à l'air et la couche extérieure (3) de la pièce avant est faite d'une matière non perméable à l'air, notamment un film plastique ou un laminé de film plastique et de nontissé.

7. Un vêtement de patient selon l'une quelconque des revendications 1-6, **caractérisé en ce que** la couche intérieure (4) s'étend sur la totalité de la couche extérieure (3).

8. Un vêtement de patient selon l'une quelconque des revendications 3-7, **caractérisé en ce que** la couche intérieure (4) est laminée sur la couche extérieure (3) à l'intérieur de la région marginale aux parties ouvrantes de la pièce avant (1).

9. Un vêtement de patient selon l'une quelconque des revendications 2-8, **caractérisé en ce que** les connexions détachables et refermables (6, 7 et 15, 16) qui relient les parties du vêtement les unes avec les autres sont formées de connexions mécaniques, préférablement de soi-disant connexions velcro.

10. Un vêtement de patient selon l'une quelconque des revendications 4, 6-9, **caractérisé en ce que** le vêtement a un arrière ouvrant.
